# EUROPEAN PATENT SPECIFICATION

(11) **EP 3 845 153 B1**
(45) Date of publication and mention of the grant of the patent: **18.12.2024**
(21) Application number: 20181567.7
(22) Date of filing: 23.06.2020
(51) Int. Cl.: A61B 17/15, A61B 17/17, A61B 17/80

(54) **SURGICAL GUIDE FOR OSTEOTOMY OPERATIONS**
CHIRURGISCHE FÜHRUNG FÜR OSTEOTOMIEOPERATIONEN
GUIDE CHIRURGICAL POUR DES OPÉRATIONS D'OSTÉOTOMIE

(30) Priority: 09.07.2019 ES 201930635
(43) Date of publication of application: 07.07.2021
(73) Proprietor: Saúde Atlântica - Gestão Hospitalar, S.A., Porto (PT)
(72) Inventor: SANCHEZ ALVAREZ, Jose Miguel, 01007 VITORIA-GASTEIZ (ES); SANCHEZ ARIZMENDIARRIETA, Xabier, 01007 VITORIA-GASTEIZ (ES); FIZ SANCHEZ, Juan Nicholas, 01004 VITORIA-GASTEIZ (ES)
(74) Representative: Couto, Cláudia

(56) References cited:
- WO-A1-2019/038240
- WO-A1-2020/037418
- ES-A1- 2 723 549
- US-A1- 2018 085 133

## Description

### TECHNICAL FIELD

The present invention refers to a surgical guide which, combined with a series of Kirschner wires, enables the correct implantation of an osteosynthesis plate during an osteotomy operation for tibial addition.

The object of the invention is to provide a device which enables an operation as precise as possible, quick, as well as safe for the surgeon, reducing the dependence on the skill of the surgeon, in other words, minimising possible human errors.

### BACKGROUND OF THE INVENTION

Alterations in the extremities are a frequent condition in the general population. It is common for them to go unnoticed or stay hidden in the diagnostic process.

Among these alterations it is worth noting alterations in the frontal or sagittal plane, i.e. deformations with respect to the normal load axis of the limb, which usually occur due to the evolution of developmental processes or as a consequence of the degeneration of the joint and the ligaments which stabilise it.

The correct analysis of these deformities requires a more extensive radiological study than simple radiology, with computed tomography (CT) usually being chosen.

Advances in computer software for processing images enable a complete analysis of the degree of correction desired.

These techniques enable virtual corrections of the alterations on the frontal or sagittal plane to be performed, giving information on the final result of a possible surgical operation.

Likewise, it enables surgical guides to be generated for intraoperative use. The guides adapt precisely to the bone surface and guide the correction of the angle in the frontal or sagittal plane at all times. Until now, said correction had to be performed by taking intraoperative visual references, these techniques being highly dependent on the observer and therefore the results of the correction not being very precise.

In this sense, invention patents EP1781219, EP1848384, EP1850782, EP1868544, EP1959878, EP2053979, EP2068771, EP2068784, EP2086434, EP2099394, EP2367485 describe a series of apparatuses, guides and plates which provide the surgeon with tools that enable them to improve the degree of exactness in the operation but that continue to pose the following problems:
- The devices and guides described by these patents require the surgeon to calculate, in the operating room, the reference point from which the guide will start to be placed, which creates the risk of human error.
- The apparatuses and guides described by these patents are made up of a large number of elements which, in order to adjust them to the requirements of the treatment of the patient, must be assembled and calibrated in the operating room, which requires due to the complexity thereof a considerable amount of time and, even more importantly, a margin of error in the calibration thereof derived from the human factor.
- In order to try to minimise this possible calibration error, the surgical operation must be performed by supervising the work with X-ray equipment, with which the hands of the surgeon are radiated for a very long period of time.
- The apparatuses and guides described by these patents require exact sequencing in the operation of the surgery, such that the drilling of the bone to obtain the holes for accommodating the osteosynthesis plate must be performed after cutting the bone, leaving the latter weakened, such that it would be desirable, at least in certain patients, to be able to carry out this operation prior to cutting the bone.

Trying to prevent the problem of calculating the reference point for the placement of an osteotomy guide, invention patent ES 2723549, of which the applicant is joint holder, describes a kit, wherein a surgical guide fundamentally participates which adapts in a personalised manner to the anatomy of the bone of the patient in the areas of interest, made in a single piece with an elongated configuration.

Although this system notably improves osteotomy operations, the configuration of the guide in a single piece makes it impossible to use it in opening osteotomy or tibial addition operations. This type of operation requires a series of additional actions to guide the cutting of the bone, for which reason the guide of ES 2723549 cannot be applied.

For its part, document US 2018/085133 A1 describes a surgical guide for osteotomy operations, constituted from a body obtained from a personalised 3D model generated by means of computerised axial tomography (CAT) or magnetic resonance of the bone to be operated on, the body having an elongated configuration, with a surface resting on the bone complementary to the physiognomy thereof with the particularity that it has a modular character, wherein three work areas are defined, an upper one, an intermediate one and a lower one, wherein an upper module, an intermediate module and a lower module interact, equipped with means for coupling and unlinking from each other, wherein the upper module includes a pair of protrusions, equipped with axial through holes, intended to guide a pair of upper Kirschner wires which are parallel to each other and the lower module has a pair of parallel protrusions equipped with axial through holes for a pair of lower Kirschner wires, parallel to each other.

Although this structuring allows a precise cut of the bone, it does not provide means for precisely preparing the bone for accommodating the osteosynthesis plate.

### DESCRIPTION OF THE INVENTION

The surgical guide for the implantation of osteosynthesis plates in opening osteotomy or tibial addition operations which is recommended solves the aforementioned problem in a completely satisfactory manner, based on a simple but effective solution.

To do so, and more specifically, the guide of the invention is made of a personalised element for each patient obtained by means of an additive manufacturing process based on a 3D model, which is generated from computerised axial tomography (CAT) or magnetic resonance, carried out in the preoperative process on the extremity of the bone to be treated, mainly the tibia, having a vertically elongated configuration, with a surface resting on the bone identical to the physical features thereof by virtue of the aforementioned process.

In accordance with the essential nature of the invention, it has been foreseen that the guide has a modular character, defining three work areas, wherein different modules participate which can be fit together and interchanged.

The upper module includes a pair of protrusions equipped with axial through holes, with an arrangement and inclination calculated beforehand customised for the specific case to be operated, such that said upper module is intended to guide a pair of parallel upper Kirschner wires, at a pre-calculated angle, with the function of maintaining said module of the osteotomy guide in the suitable and pre-calculated position during the operation.

In the intermediate area of the guide, it has been foreseen that two central modules are able to be exchanged, a first central module wherein a pair of protrusions with axial through holes are established, likewise with an arrangement and inclination calculated beforehand customised for the specific case to be operated, axial holes intended to enable a pair of intermediate parallel Kirschner wires to be guided acting as guiding means for the cutting tool in the upper plane of the bisector plane of the osteotomy.

Moreover, the participation of a second central module has been foreseen, interchangeable with the first central module, wherein a pair of holes is established with a diameter, inclination and arrangement calculated beforehand in order to enable them to guide bores which enable the osteosynthesis plate to be accommodated.

Finally, the lower module has a pair of protrusions equipped with axial through holes, with an arrangement and inclination calculated beforehand customised for the specific case to be operated, such that said lower module is intended to guide a pair of lower parallel wires with the functions of acting as a stop for said cutting tool when the cutting plane of the tibia is being obtained, as well as the function of maintaining said lower module of the osteotomy guide in the suitable and pre-calculated position during the operation.

This lower module in turn ends on the bottom in a lateral bend or arm which acts as positioning and stabilising means for the guide when it rests on an area of the bone that is smoother and easier to perform the deperiostisation. In this manner, it is not necessary to remove the periosteal tissue covering the head of the tibia, the most irregular area and where the periosteal tissue is thicker, which would need a much more complicated and intrusive operation.

In this manner, the aforementioned lateral arm wherein the lower module is angled enables a perfect positioning and immobilisation of the total assembly of the guide on the area to be treated, minimising and simplifying, as said, the deperiostisation operations.

From this structuring, it is not necessary to think or debate about the exact location of the guide or the angle to cut, since the wires used determine it, almost completely reducing the risk of human error in the placement of the guide and calibration of the same since this guide does not require calibration during the operation. This implies that with the help of the 3D models obtained in the preoperative period and the preoperative calculations supported by the computer tools, we were able to obtain a personalised osteotomy guide for each patient by means of additive manufacturing, which considerably improves the degree of accuracy of the operation and significantly reduces the time of the surgical operation, with all that it entails: less probability of infection, less time for anaesthesia, etc.

In the same manner, this modular structure makes it possible to use the intermediate modules in a different order, enabling the steps of the surgery to be reversed, i.e. the holes can be made first in order to accommodate the plate and finally insert the wires in order to guide the cut or choose the opposite order.

This change in order with respect to the initially described process enables holes to be made on uncut bones, such that, depending on the health thereof, it will enable holes to be made with less risk of breaks or fissures being produced in the bone to be treated.

Finally, it is worth noting the fact that with the guide system of the invention, since all the parameters are pre-calculated by a computer, it is not necessary to continuously supervise the work being performed by means of X-ray equipment, unnecessarily radiating the surgeon's hands.

### DESCRIPTION OF THE DRAWINGS

As a complement to the description provided below, and for the purpose of helping to make the features of the invention more readily understandable, in accordance with a practical preferred embodiment thereof, said description is accompanied by a set of plans which, by way of illustration and not limitation, represent the following:

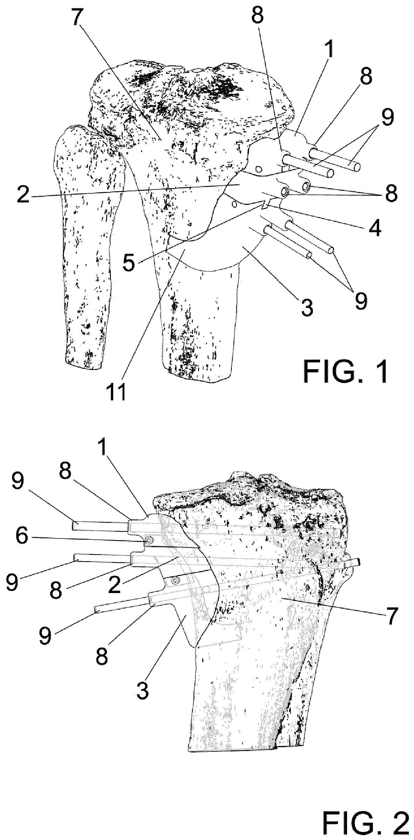

Figure 1 shows a perspective view of a surgical guide for the implantation of osteosynthesis plates in osteotomy operations carried out in accordance with the object of the present invention, duly implanted on the bone to be operated on, wherein some Kirschner wires appear as implanted, specifically the upper pair of wires and the lower pair of wires.
Figure 2 shows a profile view of the device of the invention with the Kirschner wires duly implanted.
Figure 3 shows a perspective view of the device of the invention wherein, once all the wires have been implanted, the first intermediate module is then removed.
Figure 4 shows a perspective view of the assembly of figure 3, in a later phase wherein the bone has been cut, the cut guided from above by the pair of intermediate Kirschner wires, and limited in the depth thereof by the pair of lower Kirschner wires.
Figure 5 finally shows the device with the second module implanted thereon, once the cutting phase represented in the previous figure has been completed, in order to make the corresponding holes for accommodating the osteosynthesis plate.
Figure 6 shows a side elevation view of how the treated area would look once the guide has been completely taken off.

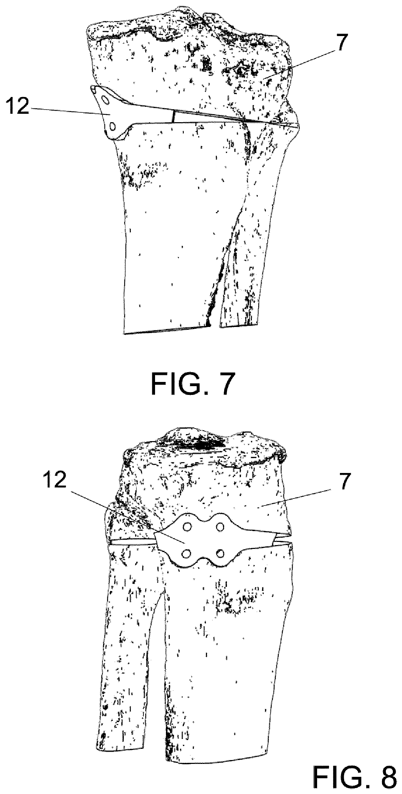

Figures 7 and 8 finally show two lateral and frontal elevation views of how the osteosynthesis plate would be arranged on the operated area.

### PREFERRED EMBODIMENT OF THE INVENTION

In light of the mentioned figures, it can be seen how the surgical guide for the implantation of osteosynthesis plates in osteotomy operations is constituted from a modular body, wherein three work areas are defined, an upper one, an intermediate one and a lower one, linked through an upper module (1), two intermediate modules (2-2') and a lower module (3), equipped with means for coupling to each other, such as complementary ribs (4) and grooves (5), lockable by means of transverse pins (6), such that these modules can be made independent when this is deemed convenient.

Moreover, the assembly formed by these modules is obtained by means of an additive manufacturing process starting from a 3D model, which is generated from computerised axial tomography (CAT), carried out in the preoperative process on the extremity of the bone (7) to be treated, having a vertically elongated configuration, with a surface resting on the bone complementary to the physical features thereof by virtue of said process.

The upper module (1) includes a pair of protrusions (8) equipped with axial through holes, with an arrangement and inclination calculated beforehand customised for the specific case to be operated, such that said upper module is intended to guide a pair of parallel upper Kirschner wires (9) the function of which is to maintain said module (1) of the osteotomy guide in the suitable and pre-calculated position during the operation.

The first central module (2) has a pair of protrusions with axial through holes, and likewise has an arrangement and inclination calculated beforehand as customised for the specific case to be operated, axial holes intended to enable a second pair of parallel Kirschner wires (9) to be guided, acting as guide means for the cutting tool in the upper plane of the bisector plane of the osteotomy.

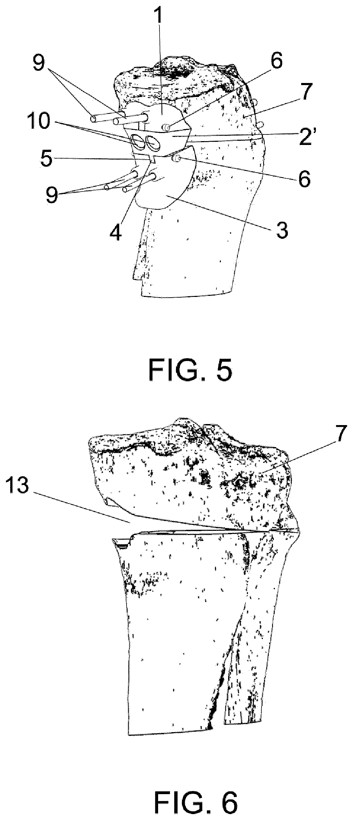

The second central module (2'), the one shown in figure 5, can be easily replaced by the first central module (2), fitting equally in the upper (1) and lower modules (3), having a pair of holes (10) with a diameter, inclination and arrangement calculated beforehand in order to enable it to act as a guide for bores which enable the osteosynthesis plate to be accommodated.

As will be explained later, the structure of the device enables different modes of operation, making it more versatile.

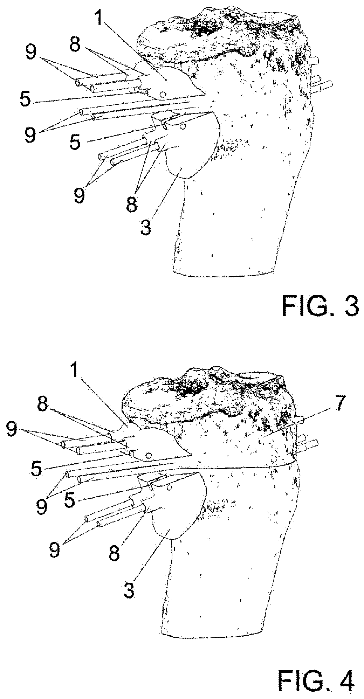

As for the lower module (3), it has a pair of protrusions (8) equipped with axial through holes, with an arrangement and inclination calculated beforehand customised for the specific case to be operated, such that said lower module is intended to guide a pair of lower parallel Kirschner wires (9) intended to act as a stop for said cutting tool when the cutting plane is being obtained, as well as the function of maintaining said lower module of the osteotomy guide in the suitable and pre-calculated position during the operation. All of this is shown in figure 4.

The lower module (3) in turn ends on the bottom in a lateral bend or arm (11) which acts as positioning and stabilising means for the entire assembly of the guide.

From this structuring, the operation, as mentioned previously, can vary depending on what the surgeon wants as far as the order of the cutting and performing holes for the adaptation of the osteosynthesis plate and is as follows:
In a process prior to surgical operation, specifically in the preoperative process, a personalised guide is prepared for the specific patient.

The protrusions (8) of each module will be provided with a different marking for the specific identification thereof and the sequence of use thereof, just like the wires (9), each of which bears a personalised identification in correspondence with marking of the respective protrusion of the guide wherein it must be placed, likewise including a depth marking, for the operative placement thereof at the exact depth.

First, the approach will be made and the bone (7) will be prepared, making an incision at the height of the osteotomy, until the operation area is opened.

Subsequently, the surgical guide is then placed with the modules thereof assembled together, adapting it to the bone, being especially applicable the arm (11) wherein the lower module (3) ends, which acts as positioning and stabilising means when resting on an area of the bone that is smoother and easy to perform the deperiostisation, stabilising the assembly formed by the three modules in the implantation thereof.

Once the guide has been stabilised, the Kirschner wires (9) are then placed, which must be inserted into the surgical guide in a certain order and position, and with a certain depth: wherein both the order and the depth of placement are defined beforehand in the Kirschner wires (9) and in the protrusions (8) of the different modules of the guide.

With the Kirschner wires (9) placed in the correct position thereof, the first intermediate module (2) is then removed. At this point, it could be interesting to cut the excess portion of the wires (9) in order to prevent different collisions between them in the successive phases.

Once the intermediate module has been removed, the cut of the osteotomy is performed, using a surgical cutting saw using the intermediate Kirschner wires as a guide and cutting until reaching the stop which establishes the contact with the lower Kirschner wires (9) and cutting limit of which is clearly perceived since the collision between the intermediate and lower Kirschner wires (9) prevents the saw from being able to continue to cut.

Next, the two intermediate wires (9) are taken out.

Subsequently, the second intermediate module (2') is placed, such that through the holes (10) thereof, bores can be made in the bone which enable the bone to be conditioned to the osteosynthesis plate.

Once these conditioning holes have been made, the guide is removed module by module and all the wires are taken out, then the space (13) of the sectioned bone is opened until the osteosynthesis plate (12) can be accommodated therein, which is fastened to the bone by means of through screws through the holes which had been drilled beforehand using as a guide the holes of the osteosynthesis plate through which the screws pass.

Then, the gap generated after opening the sectioned bone is filled by using any of the usual techniques used to do so.

Finally, the wound made is closed, with the approach used in the usual surgical procedures.

As mentioned above, the mountable and removable nature of the intermediate modules (2-2') enable the process related to cutting and making holes to be reversed, enabling the holes for accommodating the osteosynthesis plate (12) to be made first and then finally introducing the wires for guiding and limiting the cutting or opting for the order described.

## Claims

1. A surgical guide for osteotomy operations, it is constituted from a body obtained from a personalised 3D model generated by means of computerised axial tomography (CAT) or magnetic resonance of the bone to be operated on, a body which has an elongated configuration, with a surface resting on the bone complementary to the physiognomy thereof with the particularity that it has a modular character, wherein three work areas are defined, an upper one, an intermediate one and a lower one, wherein an upper module (1), a first central module (2) and a lower module (3) are configured to interact, equipped with means for coupling and unlinking from each other, such that the upper module (1) includes a pair of protrusions (8), equipped with parallel axial through holes configured to guide a pair of upper Kirschner wires (9), and the lower module (3) having a pair of parallel protrusions (8) equipped with parallel axial through holes configured to guide a pair of lower Kirschner wires (9), **characterized in that** the surgical guide further comprises a second central module (2') interchangeable with the first central module (2), wherein the first central module (2) has a pair of protrusions with parallel axial through holes configured to guide a pair of intermediate Kirschner wires (9) as guide means for a tool, the lower Kirschner wires (9) that pass through the lower module (3) constituting stop means for the cutting tool during the guiding thereof along the intermediate wires, and wherein the second central module (2') has a pair of holes acting as a guide for bores (10) configured for accommodating an osteosynthesis plate.

2. The surgical guide for osteotomy operations, according to claim 1, **characterised in that** the lower module (3) ends on the bottom in a lateral bend or arm (11) which acts as stabilisation means for the entire assembly of the guide on the bone on which deperiostisation has been performed.

3. The surgical guide for osteotomy operations, according to claim 1, **characterised in that** the coupling means between modules are made of complementary longitudinal ribs (4) and grooves (5), lockable by means of transverse pins (6).

4. The surgical guide for osteotomy operations, according to claim 1, **characterised in that** the protrusions (8) include markings with the order of insertion of the Kirschner wires (9).

5. The surgical guide for osteotomy operations, according to claims 1 and 4, **characterised in that** the Kirschner wires (9) include markings of the protrusion wherein they must be inserted and the depth at which they must be inserted.

## Patentansprüche

1. Chirurgische Führung für Osteotomieoperationen, bestehend aus einem Körper, der aus einem personalisierten 3D-Modell gewonnen wird, das mittels computergestützter Axialtomographie (CAT) oder Magnetresonanz des zu operierenden Knochens erzeugt wurde, ein Körper, der eine längliche Konfiguration aufweist mit einer Oberfläche, die auf dem Knochen aufliegt und die Physiognomie des Knochens ergänzt, mit der Besonderheit, dass sie einen modularen Charakter hat, worin drei Arbeitsbereiche definiert sind, ein oberer, ein mittlerer und ein unterer Arbeitsbereich, worin ein oberes Modul (1), ein erstes zentrales Modul (2) und ein unteres Modul (3) so konfiguriert sind, dass sie miteinander interagieren und mit Mitteln zum Ankoppeln und Abkoppeln ausgestattet sind, so dass das obere Modul (1) ein Paar von Vorsprüngen (8) aufweist, die mit parallelen axialen Durchgangslöchern ausgestattet und konfiguriert sind, um ein Paar oberer Kirschnerdrähte (9) zu führen, und das untere Modul (3) ein Paar paralleler Vorsprünge (8) aufweist, die mit parallelen axialen Durchgangslöchern ausgestattet und so ausgebildet sind, um ein Paar unterer Kirschnerdrähte (9) zu führen, **dadurch gekennzeichnet, dass** die chirurgische Führung ferner ein zweites zentrales Modul (2') umfasst, das mit dem ersten zentralen Modul (2) austauschbar ist, wobei das erste zentrale Modul (2) ein Paar Vorsprünge mit parallelen axialen Durchgangslöchern aufweist, die so ausgebildet sind, um ein Paar mittlerer Kirschnerdrähte (9) als Führungsmittel für ein Schneidwerkzeug zu führen, wobei die unteren Kirschnerdrähte (9), die durch das untere Modul (3) verlaufen, Anschlagmittel für das Schneidwerkzeug während dessen Führung entlang der mittleren Drähte umfassen und wobei das zweite zentrale Modul (2') ein Paar Löcher aufweist, die als Führung für Bohrungen (10) fungieren, die zur Aufnahme einer Osteosyntheseplatte konfiguriert sind.

2. Chirurgische Führung für Osteotomieoperationen gemäß Anspruch 1, **dadurch gekennzeichnet, dass** das untere Modul (3) auf der Unterseite in einer seitlichen Biegung oder einem Arm (11) endet, der als Stabilisierungsmittel für die gesamte Montage der Führung auf dem Knochen dient, an dem die Deperiostisierung durchgeführt wurde.

3. Chirurgische Führung für Osteotomieoperationen gemäß Anspruch 1, **dadurch gekennzeichnet, dass** die Kopplungsmittel zwischen den Modulen aus komplementären Längsrippen (4) und Nuten (5) bestehen, die durch Querstifte (6) verriegelt werden können.

4. Chirurgische Führung für Osteotomieoperationen gemäß Anspruch 1, **dadurch gekennzeichnet, dass** die Vorsprünge (8) Markierungen mit der Reihenfolge des Einführens der Kirschnerdrähte (9) enthalten.

5. Chirurgische Führung für Osteotomieoperationen gemäß den Ansprüchen 1 und 4, **dadurch gekennzeichnet, dass** die Kirschnerdrähte (9) Markierungen des Vorsprungs enthalten, in dem sie eingeführt werden müssen, sowie der Tiefe, in der sie eingesetzt werden müssen.

## Revendications

1. Guide chirurgical pour des opérations d'ostéotomie, qui est constitué d'un corps obtenu à partir d'un modèle 3D personnalisé généré au moyen d'une tomographie axiale informatisée (CAT) ou d'une résonance magnétique de l'os à opérer, un corps qui présente une configuration allongée, avec une surface reposant sur l'os complémentaire de la physionomie de celui-ci avec la particularité qu'il présente un caractère modulaire, dans lequel trois zones de travail sont définies, une supérieure, une intermédiaire et une inférieure, dans lequel un module supérieur (1), un premier module central (2) et un module inférieur (3) sont configurés pour interagir, équipés de moyens de couplage et de dissociation les uns des autres, de telle sorte que le module supérieur (1) comporte une paire de saillies (8), équipées de trous traversants axiaux parallèles configurés pour guider une paire de broches de Kirschner supérieures (9), et le module inférieur (3) ayant une paire de saillies parallèles (8) équipées de trous traversants axiaux parallèles configurés pour guider une paire de broches de Kirschner inférieures (9), **caractérisé en ce que** le guide chirurgical comprend en outre un second module central (2') interchangeable avec le premier module central (2), dans lequel le premier module central (2) présente une paire de saillies avec des trous traversants axiaux parallèles configurés pour guider une paire de broches de Kirschner intermédiaires (9) en tant que moyens de guidage pour un outil de coupe, les broches de Kirschner inférieures (9) qui traversent le module inférieur (3) constituant des moyens d'arrêt pour l'outil de coupe durant le guidage de celui-ci le long des fils intermédiaires, et dans lequel le second module central (2') présente une paire de trous servant en tant que guide pour des alésages (10) configurés pour recevoir une plaque d'ostéosynthèse.

2. Guide chirurgical pour des opérations d'ostéotomie, selon la revendication 1, **caractérisé en ce que** le module inférieur (3) se termine dans le bas en un coude ou un bras latéral (11) qui sert de moyen de stabilisation pour l'ensemble entier du guide sur l'os sur lequel la dépériostisation a été réalisée.

3. Guide chirurgical pour des opérations d'ostéotomie, selon la revendication 1, **caractérisé en ce que** les moyens de couplage entre modules sont constitués de nervures longitudinales (4) et de rainures (5) complémentaires, verrouillables au moyen de broches transversales (6).

4. Guide chirurgical pour des opérations d'ostéotomie, selon la revendication 1, **caractérisé en ce que** les saillies (8) comportent des marquages avec l'ordre d'insertion des broches de Kirschner (9).

5. Guide chirurgical pour des opérations d'ostéotomie, selon les revendications 1 et 4, **caractérisé en ce que** les broches de Kirschner (9) comportent des marquages de la saillie dans laquelle elles doivent être insérées et de la profondeur à laquelle elles doivent être insérées.
